# EUROPEAN PATENT APPLICATION

(11) **EP 3 278 728 A1**
(43) Date of publication of application: **07.02.2018**
(21) Application number: 16182116.0
(22) Date of filing: 01.08.2016
(51) Int. Cl.: A61B 5/145, A61B 5/00

(54) **AN APPARATUS, SYSTEM AND METHOD FOR DETECTING ANALYTES FROM A USER'S SKIN**

(71) Applicant: Nokia Technologies Oy, 02610 Espoo (FI)
(72) Inventor: Myyryläinen, Lea, 02360 Espoo (FI); Pohjonen, Helena, 02360 Espoo (FI)
(74) Representative: Nokia EPO representatives

(57) **Abstract**

An apparatus, method and system wherein the apparatus comprises: a pump comprising an attachment portion configured to enable the pump to be attached to a user's skin, wherein the pump is configured to draw analytes from the user's body into the apparatus by reducing pressure on the user's skin in an area covered by the pump; at least one detector configured to detect one or more analytes or combinations of analytes which are drawn into the apparatus; the apparatus being arranged to be coupled to a controller to enable the pressure on the user's skin to be adjusted.

## Description

### TECHNOLOGICAL FIELD

Examples of the disclosure relate to an apparatus, system and method for detecting analytes from a user's body. In particular, they relate to an apparatus, system and method for detecting analytes from an apparatus attached to a user's skin.

### BACKGROUND

The analysis of analytes such as volatile organic chemicals (VOCs), for instance, in a patient's breath provides an alternative, non invasive, approach for monitoring physical condition. Such techniques may be useful, for example, in early detection of cancer or other medical conditions. For instance, the detection of a particular VOC, or combinations of VOCs, or a change in the amount of a particular VOC detected, or combinations of VOCs detected may provide detailed information regarding whether cancer is present, the type of cancer present and also the progression of the cancer.

It is useful to provide an apparatus which enables improved detection and analysis of analytes such as VOCs.

### BRIEF SUMMARY

According to various, but not necessarily all, examples of the disclosure there is provided an apparatus comprising: a pump comprising an attachment portion configured to enable the pump to be attached to a user's skin, wherein the pump is configured to draw analytes from the user's body into the apparatus by reducing pressure on the user's skin in an area covered by the pump; at least one detector configured to detect one or more analytes or combinations of analytes which are drawn into the apparatus; the apparatus being arranged to be coupled to a controller to enable the pressure on the user's skin to be adjusted.

The pump may comprise a first electrode, a second electrode and a flexible electroactive polymer located between the first and second electrodes.

The pressure on the user's skin may be adjustable by controlling a voltage applied between the first and second electrodes.

The pump may comprise a plurality of first and second electrodes.

The first and second electrodes may comprise ring electrodes.

The pressure on the user's skin may be adjustable by controlling deformation of the flexible electroactive polymer.

The apparatus may be configured to enable the voltage applied across the electrodes to be manually controllable.

The apparatus may comprise a pressure sensor.

The apparatus may be configured to enable the output from the pressure sensor to be used by the controller to maintain a predefined pressure or pressure range on the user's skin.

The apparatus may comprise a chamber coupled to the pump, the chamber being configured to provide a volume into which analytes can be drawn.

A filter may be provided between the pump and the chamber, the filter being configured to remove one or more components from the analyte.

The at least one detector may comprise a sensing material configured to provide an electrical output in response to the presence of an analyte or mixture of analytes.

The electrical output may be characteristic of a particular analyte or mixture of analytes.

The at least one detector may be provided within the pump and/or the chamber.

The at least one detector may comprise at least one of: surface acoustic wave sensors, bulk acoustic wave sensors, film acoustic resonator based detectors, quartz crystal microbalance, chemiresistors based on metal oxides, metal oxide semiconductors, conducting polymers, optical techniques such as fluorescence image and infra red, nanosensors comprising carbon nanotubes (CNTs) or graphene, or nanosensors coated with one or more DNA oligomer sequences, which DNA oligomer sequences may be the same or different.

A plurality of detectors may be provided. The plurality of detectors may be configured to detect different analytes.

The apparatus may comprise an interface configured to enable the apparatus to be coupled to at least one remote detector.

The at least one remote detector may comprise at least one of: mass spectrometer, ion mobility spectrometer, gas sensor, flame ionization detector, or headspace solid phase microextraction apparatus.

The at least one remote detector may comprise a separator configured to separate the one or more analytes or combinations of analytes into different components.

The separator may comprise gas or liquid chromatography.

The apparatus may comprise at least one biosensor arranged to provide biometric parameters of the user.

The apparatus may comprise a transceiver arranged to enable the apparatus to communicate with a remote device.

The apparatus may comprise a band wearable on a user's arm or wrist.

The apparatus may comprise a display, the display being configured to display information.

According to various, but not necessarily all, examples of the disclosure there is provided a system comprising an apparatus according to any of the preceding paragraphs and a controller, the controller being configured to enable the pressure on the user's skin to be adjusted.

According to various, but not necessarily all, examples of the disclosure there is provided a method comprising: providing an apparatus comprising a pump, the pump comprising an attachment portion configured to enable the pump to be attached to a user's skin, wherein the pump is configured to draw analytes from the user's body into the apparatus by reducing pressure on the user's skin in an area covered by the pump; providing at least one detector configured to detect one or more analytes or combinations of analytes which are drawn into the apparatus; the apparatus being arranged to be coupled to a controller to enable the pressure on the user's skin to be adjusted.

The pump may comprise a first electrode, a second electrode and a flexible electroactive polymer located between the first and second electrodes.

The pressure on the user's skin may be adjustable by controlling a voltage applied between the first and second electrodes.

The pump may comprise a plurality of first and second electrodes.

The first and second electrodes may comprise ring electrodes.

The pressure on the user's skin may be adjustable by controlling deformation of the flexible electroactive polymer.

The voltage applied across the electrodes may be manually controllable.

The method may comprise providing a pressure sensor.

The output from the pressure sensor may be used by the controller to maintain a predefined pressure or pressure range on the user's skin.

The method may comprise providing a chamber coupled to the pump, the chamber being configured to provide a volume into which analytes can be drawn.

The method may comprise providing a filter between the pump and the chamber, the filter being configured to remove one or more components from the analyte.

The at least one detector may comprise a sensing material configured to provide an electrical output in response to the presence of an analyte or mixture of analytes.

The electrical output may be characteristic of a particular analyte or mixture of analytes.

The at least one detector may be provided within the pump and/or the chamber.

The at least one detector may comprise at least one of: surface acoustic wave sensors, bulk acoustic wave sensors, film acoustic resonator based detectors, quartz crystal microbalance, chemiresistors based on metal oxides, metal oxide semiconductors, conducting polymers, optical techniques such as fluorescence image and infra red, nanosensors comprising carbon nanotubes (CNTs) or graphene, or nanosensors coated with one or more DNA oligomer sequences, which DNA oligomer sequences may be the same or different.

The method may comprise providing a plurality of detectors. The plurality of detectors may be configured to detect different analytes.

The method may comprise providing an interface configured to enable the apparatus to be coupled to at least one remote detector.

The at least one remote detector may comprise at least one of: mass spectrometer, ion mobility spectrometer, gas sensor, flame ionization detector, or headspace solid phase microextraction apparatus.

The at least one remote detector may comprise a separator configured to separate the one or more analytes or combinations of analytes into different components.

The separator may comprise gas or liquid chromatography.

The method may comprise providing at least one biosensor arranged to provide biometric parameters of the user.

The method may comprise providing a transceiver arranged to enable the apparatus to communicate with a remote device.

The method may comprise providing a band wearable on a user's arm or wrist.

The method may comprise providing a display, the display being configured to display information.

The method may comprise providing a system, the system comprising an apparatus according to any of the preceding paragraphs and a controller, the controller being configured to enable the pressure on the user's skin to be adjusted.

According to various, but not necessarily all, examples of the disclosure there may be provided examples as claimed in the appended claims.

### BRIEF DESCRIPTION

For a better understanding of various examples that are useful for understanding the detailed description, reference will now be made by way of example only to the accompanying drawings in which:
Fig. 1 illustrates an apparatus;
Fig. 2 illustrates an example pump in a first condition;
Fig. 3 illustrates the example pump of Fig. 2 in a second condition;
Fig. 4 illustrates an example detector;
Fig. 5 illustrates another example apparatus;
Fig. 6 illustrates another example apparatus;
Fig. 7 illustrates another example apparatus;
Fig. 8 illustrates another example apparatus;
Fig. 9 illustrates another example apparatus;
Fig. 10 illustrates another example apparatus;
Fig. 11 illustrates a system comprising an apparatus;
Fig. 12 illustrates a method.

### DETAILED DESCRIPTION

The Figures illustrate an apparatus 1 comprising: a pump 3 comprising an attachment portion 5 configured to enable the pump 3 to be attached to a user's skin, wherein the pump 3 is configured to draw analytes from the user's body into the apparatus 1 by reducing pressure on the user's skin in an area covered by the pump 3; and at least one detector 7 configured to detect one or more analytes or combinations of analytes which are drawn into the apparatus 1; the apparatus 1 being arranged to be coupled to a controller 103 to enable the pressure on the user's skin to be adjusted.

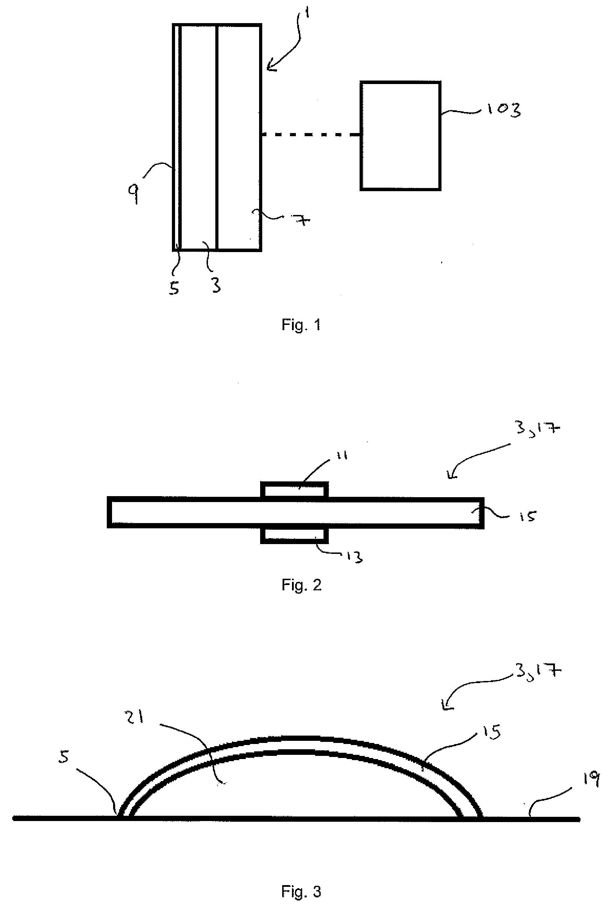

Fig. 1 schematically illustrates an apparatus 1 according to examples of the disclosure. The example apparatus 1 comprises a pump 3 and at least one detector 7. The pump 3 comprises an attachment portion 5 configured to enable the pump 3 to be attached to a user's skin. The apparatus 1 is arranged to be coupled to a controller 103 to enable the pressure on the user's skin to be adjusted.

The pump 3 comprises any means which may be configured to draw analytes from the user's body into the apparatus 1 by reducing pressure on the user's skin in the area covered by the pump 3. The analytes may therefore be actively drawn from a user's body into the apparatus 1. The parts of the body from which analytes are drawn may be the skin, or the tissue underlying the skin. Analytes could also be drawn from the surface of the user's skin. Accordingly, the pump 3 may comprises any means which may be configured to draw analytes from the surface of the user's skin, the user's skin or tissue underlying the user's skin into the apparatus 1 by reducing pressure on the user's skin in the area covered by the pump 3.

The pump 3 may comprise any device which moves fluids by a mechanical action. The pump may be a vacuum pump. Fluids may be liquids, gases or slurries. The fluids may comprise the analytes of interest.

The apparatus 1 may also be configured such that the temperature of a user's skin can be controlled in the area covered by the pump 3.

The attachment portion 5 comprises any means which may be configured to enable the pump 3 to be attached to a user's skin. The attachment portion 5 may be any portion of the pump which enables the pump to be reversibly attached to the user's skin. The attachment portion 5 may comprise an abutment surface 9 which is engageable against a user's skin. The abutment surface 9 may have adhesive properties, or an adhesive, such as glue, may be applied between the abutment surface 9 and the user's skin. The abutment surface 9 may be pressed against a user's skin during use of the pump 3. The abutment surface 9 may be urged against a user's skin during use of the pump 3 as the pressure is reduced.

The at least one detector 7 comprises any means which may be configured to detect one or more analytes or combinations of analytes which are drawn into the apparatus 1. The at least one detector 7 may be any device capable of detecting the presence of a particular analyte or combination of analytes. The at least one detector 7 may be configured to provide an indication that a particular analyte or mixture of analytes is present. The at least one detector 7 may provide an electrical output which is characteristic of a particular analyte or mixture of analytes. The at least one detector 7 may provide a visual output which is characteristic of a particular analyte or mixture of analytes, for instance a colour change.

The apparatus 1 is arranged to be coupled to a controller 103 to enable the pressure on the user's skin to be adjusted. The apparatus 1 may be arranged to be coupled to a controller 103 to enable the pressure on the user's skin to be adjusted manually or automatically, for instance, to maintain a predetermined pressure or pressure range, or to maintain a pressure or pressure range which is comfortable to the user. The pressure on the user's skin may be adjusted manually in response to a user input which may be made via a user interface or another suitable means. The pressure on the user's skin may be adjusted automatically in response to a control signal from the pump 3 without any other input from the user.

The predetermined pressure or pressure range may be a range which is comfortable to the user, or at which particular analytes of interest are drawn from the user's body. For example the analytes could be drawn from the surface of the skin, the skin or tissue underlying the skin. The apparatus may be arranged such that the pressure on a user's skin can be reduced gradually such that analytes on the surface of the skin may first be drawn into the apparatus 1, followed by analytes from the skin, followed by analytes from the underlying tissue.

Maintenance of a predetermined pressure or pressure range, or maintenance of a pressure or pressure range which is comfortable to the user may be by way of a feedback loop. An example feedback loop is determining that the reduced pressure on the user's skin is below a predetermined level and subsequently increasing the pressure to the predetermined level. A further example feedback loop is determining that the reduced pressure on the user's skin is above a predetermined level and subsequently further reducing the pressure to the predetermined level. A yet further example feedback loop is determining that the reduced pressure on the user's skin is uncomfortable to the user and subsequently increasing the pressure until the user is comfortable.

Figs. 2 and 3 schematically illustrate a pump 3 according to examples of the disclosure in a first and second condition respectively. The pump 3 may comprise a first electrode 11, a second electrode 13 and a flexible electroactive polymer 15 located between the first and second electrodes 11, 13. This type of pump 3 is referred to as an electroactive polymer pump 17 in the rest of the description.

The first and second electrodes 11, 13 may be compliant electrodes. The first and second electrodes 11, 13 may have a uniform thickness. The first and second electrodes 11, 13 may be circular in plan view, and may comprise ring electrodes. The pump may comprise a plurality of first and second electrodes 11, 13. The first and second electrodes 11, 13 may comprise any suitable material which may function as an electrode, for instance, carbon black, carbon nanoparticles, carbon nanotubes, graphene particles etc. The flexible electroactive polymer 15 may be coated on a first side with a layer of first electrodes 11 and on a second side with a layer of second electrodes 13.

The flexible electroactive polymer 15 may be an ionic or dielectric electroactive polymer.

The electroactive polymer 15 is coupled to the first and second electrodes 11, 13 so that the application of a voltage between the first and second electrodes 11, 13 may cause deformation of the flexible electroactive polymer 15. The application of a voltage between the first and second electrodes 11, 13 may create an electrostatic force which brings the first and second electrodes 11, 13 closer together and flattens the flexible electroactive polymer 15 thereby causing the flexible electroactive polymer 15 to deform.

In some examples, the deformation may be from a first condition as shown in Fig. 2 to a second condition as shown in Fig. 3. In other examples, the flexible electroactive polymer pump 17 may be arranged such that it adopts different sizes and/or shapes. The degree of deformation may be controlled by varying the voltage between the first and second electrodes 11, 13.

The electroactive polymer 15 may deform away from the user's skin 19 where it is not attached to the user's skin 19 by the attachment portion 5. Deformation of the electroactive polymer 15 away from the user's skin 19 may increase the volume of an area 21 defined between the electroactive polymer 15 and the user's skin 19. Increasing the volume of the area 21 defined between the electroactive polymer 15 and the user's skin 19 may decrease the pressure on the user's skin 19 in the defined area 21 such that analytes are drawn into the apparatus 1.

The pressure on the user's skin may be adjustable by controlling the voltage applied between the first and second electrodes 11, 13 since the degree of deformation of the electroactive polymer 15, and hence the volume of the area 21 defined between the electroactive polymer 15 and the user's skin 19, is proportional to the voltage.

The apparatus 1 may be arranged to be coupled to a controller 103 to enable the voltage applied across the electrodes 11, 13 to be adjusted manually or automatically, for instance, to maintain a predetermined pressure or pressure range or to maintain a pressure or pressure range which is comfortable to the user.

The electroactive polymer 15 may have a thickness which enables the electroactive polymer 15 to be easily deformed. In some examples the electroactive polymer 15 may have a thickness of around 0.1 to 1.0 mm, and may have a thickness of around 0.2 to 0.5 mm, and may have a thickness of 0.3mm.

The electroactive polymer 15 may be substantially circular which enables the electroactive polymer 15 to be uniformly deformed. In some examples the electroactive polymer 15 may have a radius which is suitable for it to be placed in a device which is worn by a user. In some examples the electroactive polymer 15 may have a radius of around 5 to 25 mm, and may have a radius of around 15 to 20 mm, and may have a radius of around 18.5 mm.

The electroactive polymer 15 may be configured to deform by an amount sufficient to reduce the pressure on a user's skin to the required level. In some examples electroactive polymer 15 may be configured to deform by around 200 to 600 %, and may be configured to deform by around 350 to 500 %, and may be configured to deform by around 440 %.

The electroactive polymer pump 17 may have a mass appropriate for in to be incorporated into a device worn by a user. In some examples electroactive polymer pump 17 may have a mass of around 5 to 20 g, and may have a mass of around 10 to 15 g, and may have a mass of around 12.45 g.

The voltage applied between the first and second electrodes 11, 13 may be around 200 to 3950 V, the delta voltage may be around 200 to 400 V, and the slew rate may be around 139 to 319 kV/s. The voltage, delta voltage and slew rate may be selected to provide optimum functioning of the electroactive polymer pump 17.

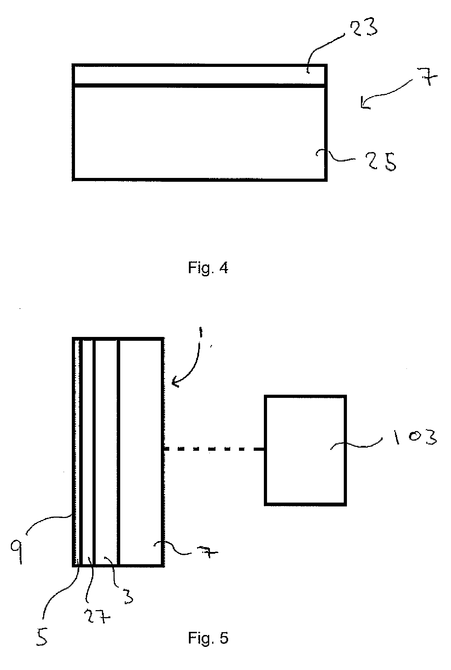

Fig. 4 schematically illustrates a detector 7 according to examples of the disclosure.

The at least one detector 7 may comprise a sensing material 23 and a substrate 25, the detector 7 may comprise any means configured to provide an electrical output in response to the presence of an analyte or mixture of analytes. The electrical output may be characteristic of a particular analyte or mixture of analytes. The sensing material may be configured to interact with the analyte such that a change in a chemical or physical property of the sensing material 23 is induced which is transduced into an electrical output.

The analytes from the user's skin may be odorants, which odorants may comprise volatile organic chemicals. The volatile organic chemicals may comprise at least one of: hydrocarbons, alcohols, ketones, aldehydes, aromatics, pyrroles or carboxylic acids. The hydrocarbons may comprise at least one of: 2,4-dimethyl-1-heptene, 2-xylene, 2,3-dimethylhexane and 2,2-dimethylbutane. The alcohol may comprise 2-ethylhexanol, cyclohexanol or isoamyl alcohol. The ketones may comprise at least one of: 2-nonanone, 2-dodecanone or 4-methyl-2-heptanone. The aldehydes may comprise benzaldehyde. The aromatics may comprise 1,3-dis-ter-butylbenzene. The pyrroles may comprise pyrrolodine. The carboxylic acids may comprise isovaleric acid or isobutyric acid. Any other suitable odorants may be used in examples of the disclosure.

The at least one detector 7 may comprise at least one of: surface acoustic wave sensors, bulk acoustic wave sensors, film acoustic resonator based detectors, quartz crystal microbalance, chemiresistors based on metal oxides, metal oxide semiconductors, conducting polymers, optical techniques such as fluorescence image and infra red, nanosensors comprising carbon nanotubes (CNTs) or graphene, or nanosensors coated with one or more DNA oligomer sequences, which DNA oligomer sequences may be the same or different. Any other suitable detectors may be used in examples of the disclosure.

The at least one detector 7 may be arranged to enable multiple different detection techniques, such as those described in the above paragraph.

The at least one detector 7 may comprises at least one electronic nose. An electronic nose may be understood as a device capable of detecting analtyes, such as odorants. An electronic nose may be understood to comprise a mechanism for analyte detection, such as an array of electronic sensors, and a mechanism for pattern recognition, such as a neural network.

The detector 7 may be capable of detecting analytes which are present in a concentration below one part per million, and may be capable of detecting analytes which are present in a concentration below one part per billion, and may be capable of detecting analytes which are present in a concentration below one part per trillion.

Fig. 5 schematically illustrates another apparatus 1 according to examples of the disclosure. Many features of the apparatus 1 of Fig. 5 are similar to those previously described, and where features are the same or similar the same reference numerals have been used and these features will not be described again for the sake of brevity. The example of Fig. 5 comprises an apparatus 1 further comprising a pressure sensor 27.

The apparatus 1 is configured to enable the output from the pressure sensor 27 to be used by the controller 103 to maintain a predefined pressure or pressure range on the user's skin. The pressure sensor 27 may act as a transducer, and may generate an electrical signal as a function of the pressure imposed. The apparatus 1 may be arranged to be coupled to a controller 103 to enable the pressure on the user's skin to be adjusted manually or automatically in response to the pressure imposed on the pressure sensor, for instance, to maintain a predetermined pressure or pressure range.

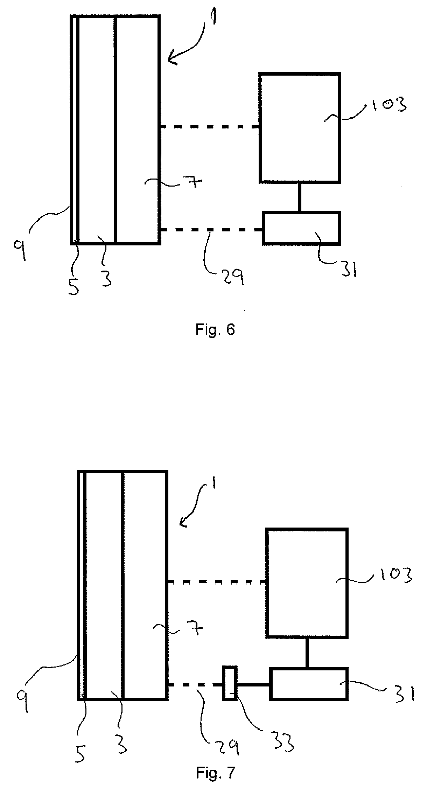

Fig. 6 schematically illustrates another apparatus 1 according to examples of the disclosure. The example of Fig. 6 comprises an apparatus 1 further comprising an interface 29 configured to enable the apparatus 1 to be coupled to at least one remote detector 31.

The interface 29 may be a conduit to enable analytes to be transferred from the apparatus 1 to the at least one remote detector 31. The conduit 29 may be a tube or pipeline, and may comprise means to urge the analytes from the apparatus 1 to the at least one remote detector 31.

The at least one remote detector 31 may comprise at least one of: mass spectrometer, ion mobility spectrometer, gas sensor, flame ionization detector, or headspace solid phase microextraction apparatus, or any other suitable detector.

The at least one remote detector 31 may be capable of detecting analytes which are present in a concentration below one part per million, and may be capable of detecting analytes which are present in a concentration below one part per billion, and may be capable of detecting analytes which are present in a concentration below one part per trillion. The at least one remote detector 31 may be more sensitive than the at least one detector 7.

Fig. 7 schematically illustrates another apparatus 1 according to examples of the disclosure. The example of Fig. 7 comprises an apparatus 1 further comprising a separator 33 configured to separate the one or more analytes or combinations of analytes into different components. The separator 33 may comprise means for enabling gas or liquid chromatography, or any other suitable separation technique.

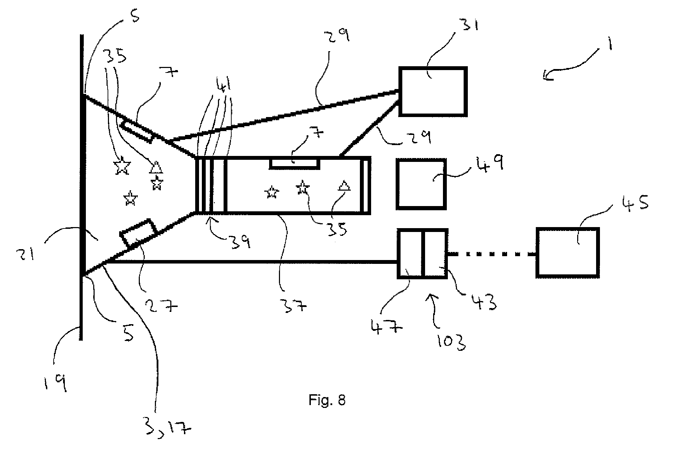

Fig. 8 schematically illustrates another apparatus 1 according to examples of the disclosure. Many features of the apparatus 1 of Fig. 8 are similar to those previously described, and where features are the same or similar the same reference numerals have been used and these features will not be described again for the sake of brevity.

The apparatus 1 of Fig. 8 is shown in use with analytes 35 being drawn from the surface of the user's skin 19, the user skin or tissue underlying the user's skin 19.

The apparatus 1 may comprise a chamber 37 coupled to the pump 3. The chamber 37 may be provided to enable further detectors 7 to be provided in the apparatus 1, which detectors 7 may be separate from any detectors 7 located in the pump 3. A filter 39 may be provided between the pump 3 and the chamber 37. The filter 39 may be provided to remove unwanted components from the analyte. The pump may be an electroactive polymer pump 17 as previously described. The filter 39 may have one or more layers 41, and each layer 41 may be configured to filter a different component of the analyte 35. The filter 39 may, for instance, be configured to filter a particular component or components from the analyte 35, which component or components may be gas or liquid. An example of a component which may be filtered from the anlayte 35 is the moisture component.

The apparatus 1 may comprise a plurality of detectors 7. The plurality of detectors may be configured to detect different analytes 35. In the example shown in Fig. 8, one detector 7 is provided in the pump 3 and two detectors 7 are provided in the chamber 37. Other variations are possible. For example, the at least one detector 7 may be provided just in the chamber 37, or just in the pump 3, or a plurality of detectors 7 may be provided in both the pump 3 and the chamber 37. The detectors 7 may be arranged to be coupled to the controller 103. The detectors 7 may be coupled wirelessly or by wires.

The apparatus 1 may comprise a pressure sensor 27 within the pump 3. The pressure sensor 27 may be arranged to be coupled to the controller 103, and may be coupled wirelessly or by wires.

An interface 29 in the form of a conduit may be provided to enable analytes 35 to be transferred from the pump 3 and/or the chamber 37 to the at least one remote detector 31. In the example shown in Fig. 8, an interface 29 in the form of a conduit is provided both between the pump 3 and the at least one remote detector 31 and between the chamber 37 and the at least one remote detector 31.

The controller 103 may comprise a transceiver 43 arranged to enable the controller 103 to communicate with a remote device 45. The controller 103 may communicate with the remote device 45 wirelessly or by wires. The controller 103 may be configured to upload or download content from the internet. The remote device 45 may enable more detailed analysis of the data, and comparisons with existing data sets. The remote device 45 may be configured to identify trends in historic data sets.

The controller 103 may also comprise a processor 47, which may comprise an electronic signal conditioner. The processor may also comprise an analogue to digital converter to convert an analogue electrical signal to a digital signal. The processor 47 may receive analogue electrical signals from the at least one detector 7, the at least one remote detector 31, and the pressure sensor 27, and these analogue electrical signals may be converted to digital signals by the analogue to digital converter. The processor 47 may be configured to apply pattern recognition techniques to the analogue or digital signals to analyse the data included in the analogue or digital signals to detect, classify or identify analytes.

The apparatus may comprise a display 49, and the display 49 may be coupled wirelessly or by wires to the controller 103.

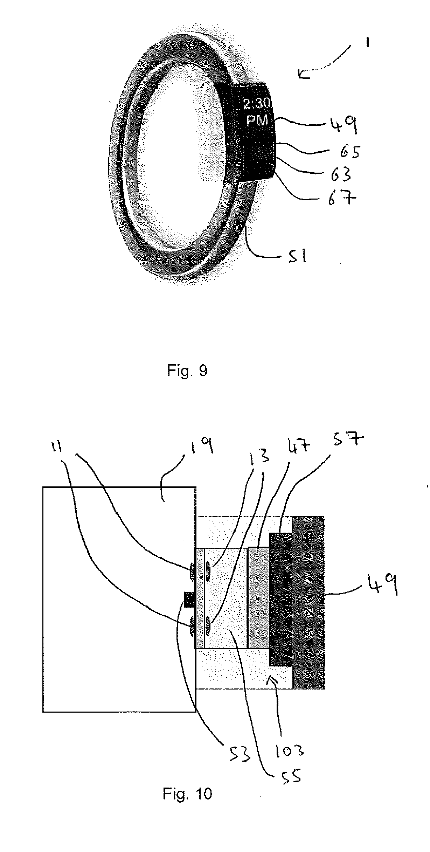

Fig. 9 shows another apparatus 1 according to examples of the disclosure. The apparatus 1 of Fig. 9 may further comprise a band 51 wearable on a user's arm, wrist or leg. The band 51 may be in the form of a wrist watch or other device, which device may be portable. The apparatus 1 may be removable from the band 51. The apparatus may be locatable in different bands 51. The different bands 51 may be configured to be wearable about different parts of a user's body, for example, a user's wrist, arm, leg or torso.

The band 51 may be arranged to push the pump 3, and particularly the attachment portion 5 into contact with the user's skin. The band 51 may be flexible. The band 51 may comprise fastening means. The display 49 of the apparatus 1 of Fig. 9 is showing the time, but could show any information, for instance, analysis results and alerts.

The apparatus of Fig. 9 also comprises a user interface which enables a user to make user selections. In the example of Fig. 9, the user interface comprises an operating panel 63, which may comprise a touch screen 67. The operating panel 63 may comprise user selectable option 65. The touch screen may be arranged to detect user inputs such as swipes, pressure or any other suitable input.

The operating panel 63 may enable manual operation of the apparatus 1. The apparatus 1 may be programmable. The apparatus 1 may be programmable via the operating panel 63. The apparatus 1 may be programmable to operate according to a predefined schedule. The apparatus 1 may be programmable to operate according to any appropriate parameters, such as location, time of day, user biometric data, or combinations of different parameters.

The apparatus 1 may be arranged to be remotely operable. The apparatus 1 may be configured such that data can be retrieved from the apparatus 1 by a remote electronic device. The retrieval of data may be by an authorised third party. The retrieved data may be used by a remote electronic device to determine the efficacy of a treatment regime.

Fig. 10 schematically illustrates the apparatus 1 of Fig. 9. Corresponding reference numerals are used for corresponding features. The apparatus 1 may comprise at least one biosensor 53 arranged to provide biometric parameters of the user. The biometric parameters may be heart rate, or heart rate variability, or any other suitable parameters. The first and second electrodes 11, 13 may also be configured to provide biometric parameters of the user.

The apparatus 1 may comprise an electroactive polymer pump 17 as previously described comprising first and second electrodes 11, 13 and a flexible electroactive polymer 15 located between the first and second electrodes 11, 13. In this case, two first electrodes 11 and two second electrodes 13 are provided. The electroactive polymer pump 17 comprises an air cavity 55 coupled to the electroactive polymer 15.

In use, the application of a voltage between the first and second electrodes 11, 13 causes the electroactive polymer 15 to deform away from the user's skin 19 where it is not attached to the user's skin 19 by the attachment portion 5. Deformation of the electroactive polymer 15 away from the user's skin 19 (as shown schematically in Fig. 3) increases the volume of the area 21 defined between the electroactive polymer 15 and the user's skin 19 and decreases the volume of the air cavity 55. An increase in the volume of the area 21 may reduce pressure on the user's skin. A decrease in the volume of the air cavity 55 may increase pressure in the air cavity 55.

The apparatus 1 may be arranged to be coupled to a controller 103, the controller 103 comprising electronics 57 and the processor 47. The electronics 57 may comprise a power source, such as batteries and the transceiver 43. The electronics 57 and power source may be shared with any device to which the apparatus 1 is coupled.

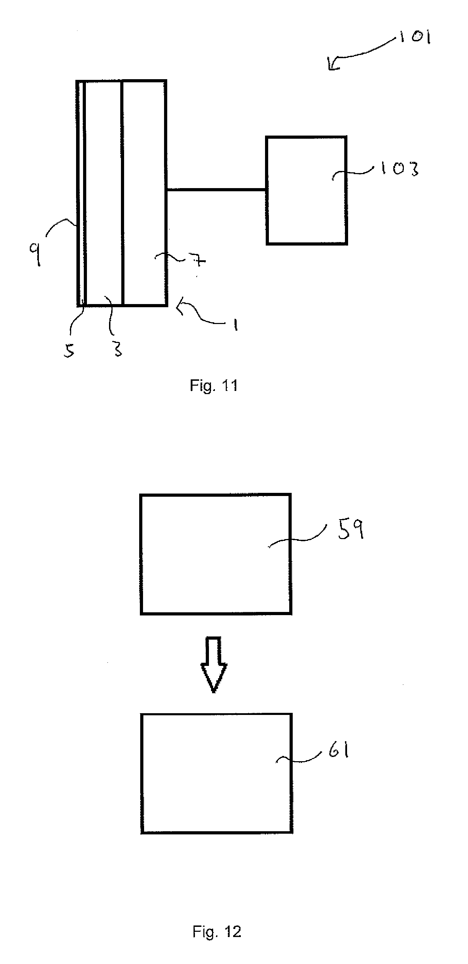

Fig. 11 illustrates a system 101 according to examples of the disclosure. The system 101 comprises an apparatus 1 as previously described and a controller 103 as previously described.

Fig. 12 illustrates a method according to examples of the disclosure. At block 59 the method comprises providing an apparatus 1 comprising a pump 3, the pump 3 comprising an attachment portion 5 configured to enable the pump 3 to be attached to a user's skin 19, wherein the pump 3 is configured to draw analytes from the user's body into the apparatus by reducing pressure on the user's skin 19 in an area covered by the pump 3. At block 61 the method comprises providing at least one detector 7 configured to detect one or more analytes or combinations of analytes which are drawn into the apparatus 1; the apparatus 1 being arranged to be coupled to a controller 103 to enable the pressure on the user's skin 19 to be adjusted.

The method may comprise any of the steps described in any of the preceding statements or description.

Examples of the disclosure provide an apparatus which enables the detection of analytes from a user's skin. In some examples, this may allow for early detection of cancer or other physical conditions. The detection of a particular analyte, or combinations of analytes, or a change in the amount of a particular analyte detected, or combinations of analytes detected may provide detailed information regarding whether cancer is present, the type of cancer and also the progression of the cancer.

In some examples the apparatus 1 may be arranged such that a predetermined pressure or pressure range, or pressure or pressure range which is comfortable to the user may be maintained, and this may be by way of a feedback loop. Accordingly, damage to the user's skin and underlying tissue can be avoided. User discomfort when using the apparatus can be avoided. Furthermore, the pressure can be adjusted such that analytes can be drawn from just the surface of the skin, the user's skin or also from the underlying tissue, or first from the surface of the skin and subsequently from the skin followed by the underlying tissue. Other environmental parameters in the area covered by the pump 3, such as temperature, can also be controlled to facilitate the drawing of analytes from the user's body and subsequent detection of the analytes.

In some examples a filter 39 may be provided between the pump 3 and chamber 37. This may improve analyte detection by removing components of the analyte which may reduce the sensitivity of the detector, such as moisture.

The provision of at least one detector 7 in the pump 3 may provide rapid detection of analytes.

In some examples detectors 7 may be capable of real time detection such that data concerning the presence of analytes of interest, and thus detection of cancer or other physical conditions, can be generated immediately.

In some examples detectors 7 may comprise nanosensors such as carbon nanotubes (CNTs) and graphene, and particularly nanosensors coated with one or more DNA oligomer sequences. These may be highly sensitive and may allow a high degree of specificity in analyte detection such that it is possible to distinguish between different similar anlaytes such as enantiomers of chiral compounds.

In some examples the provision of a transceiver 43 may enable a third party, such as a medical professional, to access the apparatus 1 to trigger analysis, to set protocols or to retrieve data.

In some examples the provision of an electroactive polymer pump 17 may enable the apparatus 1 to operate silently. This may be particularly beneficial if the apparatus 1 is comprised in a personal device such as a wrist band, arm band or watch.

In some examples the electrodes 11, 13 may comprise ring electrodes. This may provide optimum deformation of the electroactive polymer 15 and therefore optimum functioning of the electroactive polymer pump 17.

In some examples the provision of display 49 may enable immediate visualisation of analytical data and other information.

The term "comprise" is used in this document with an inclusive not an exclusive meaning. That is any reference to X comprising Y indicates that X may comprise only one Y or may comprise more than one Y. If it is intended to use "comprise" with an exclusive meaning then it will be made clear in the context by referring to "comprising only one..." or by using "consisting".

In this brief description, reference has been made to various examples. The description of features or functions in relation to an example indicates that those features or functions are present in that example. The use of the term "example" or "for example" or "may" in the text denotes, whether explicitly stated or not, that such features or functions are present in at least the described example, whether described as an example or not, and that they can be, but are not necessarily, present in some of or all other examples. Thus "example", "for example" or "may" refers to a particular instance in a class of examples. A property of the instance can be a property of only that instance or a property of the class or a property of a subclass of the class that includes some but not all of the instances in the class. It is therefore implicitly disclosed that a features described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. An apparatus comprising:
a pump comprising an attachment portion configured to enable the pump to be attached to a user's skin, wherein the pump is configured to draw analytes from the user's body into the apparatus by reducing pressure on the user's skin in an area covered by the pump;
at least one detector configured to detect one or more analytes or combinations of analytes which are drawn into the apparatus;
the apparatus being arranged to be coupled to a controller to enable the pressure on the user's skin to be adjusted.

2. An apparatus according to any of the preceding claims, in which the pump comprises a first electrode, a second electrode and a flexible electroactive polymer located between the first and second electrodes.

3. An apparatus according to claim 2, in which the pressure on the user's skin is adjustable by controlling a voltage applied between the first and second electrodes.

4. An apparatus according to any of the preceding claims, in which the apparatus comprises a pressure sensor.

5. An apparatus according to claim 4, in which the apparatus is configured to enable the output from the pressure sensor to be used by the controller to maintain a predefined pressure or pressure range on the user's skin.

6. An apparatus according to any of the preceding claims, in which the apparatus comprises a chamber coupled to the pump, the chamber being configured to provide a volume into which analytes can be drawn.

7. An apparatus according to claim 6, in which a filter is provided between the pump and the chamber, the filter being configured to remove one or more components from the analyte.

8. An apparatus according to any of the preceding claims, in which the at least one detector comprises a sensing material configured to provide an electrical output in response to the presence of an analyte or mixture of analytes.

9. An apparatus according to any of the preceding claims, in which the apparatus comprises an interface configured to enable the apparatus to be coupled to at least one remote detector.

10. An apparatus according to claim 9, in which the at least one remote detector comprises a separator configured to separate the one or more analytes or combinations of analytes into different components.

11. An apparatus according to any of the preceding claims, in which the apparatus comprises a transceiver arranged to enable the apparatus to communicate with a remote device.

12. An apparatus according to any of the preceding claims, in which the apparatus comprises a display, the display being configured to display information.

13. An apparatus according to any of the preceding claims, in which the apparatus comprises a band wearable on a user's arm or wrist.

14. A system comprising an apparatus according to any of the preceding claims and a controller, the controller being configured to enable the pressure on the user's skin to be adjusted.

15. A method comprising:
providing an apparatus comprising a pump, the pump comprising an attachment portion configured to enable the pump to be attached to a user's skin, wherein the pump is configured to draw analytes from the user's body into the apparatus by reducing pressure on the user's skin in an area covered by the pump;
providing at least one detector configured to detect one or more analytes or combinations of analytes which are drawn into the apparatus;
the apparatus being arranged to be coupled to a controller to enable the pressure on the user's skin to be adjusted.
